# EUROPEAN PATENT APPLICATION

(11) **EP 1 343 010 A1**
(43) Date of publication of application: **10.09.2003**
(21) Application number: 02075857.9
(22) Date of filing: 04.03.2002
(51) Int. Cl.: G01N 33/543

(54) **Matrix for coupling of a biological molecule, method for producing and use of said matrix**

(71) Applicant: Glaucus Proteomics B.V., 3984 NZ Odijk (NL)
(72) Inventor: Wischerhoff, Erik Heinz, 3981 ZK Bunnik (NL)
(74) Representative: Prins, Adrianus Willem

(57) **Abstract**

The present invention provides a special matrix for coupling a biological molecule thereto. The matrix comprises essentially only charges on the leaving groups thereby allowing for a matrix having coupled biological molecule, wherein the matrix does essentially not comprise a charge other than possibly on the biological molecule. This feature allows for the production of microarrays that have a very robust behaviour over a broad range of biological conditions tested.

## Description

The invention relates to the field of surface chemistry and more in particular to the field of surfaces for reacting biological molecules thereto.

The study of interactions of biological molecules such as protein-antibody or peptide-antibody interactions has many applications in biology and medicine. To perform those studies, it is often necessary to immobilise one of the interacting species.

In recent years much attention has been given to mass screening assays wherein a large number of potential interactions are being screened simultaneously. Surfaces comprising many immobilisation matrices are typically chosen for these analyses, if not for anything else then to facilitate handling of the many samples to be tested.

Although the theory is simple, it is not straightforward to actually develop properly functioning immobilizing matrices. Problems are often encountered when the matrix is contacted with test samples comprising potentially interacting molecules. At least part of the problem lies in the fact that, in case of microarrays comprising a plurality of matrices, many different interactions are being analysed at once. Considering the large variety of biological molecules on the array and of the molecules to be tested, conditions must be chosen such that non-specific (background) interactions are sufficiently low for all the studied interactions. This is of specific importance when screening samples containing proteins in a complex mixture, e. g. body fluids, for protein-protein or antibody-protein interactions. This requirement for low non-specific interactions over a broad range of conditions poses a technical challenge of considerable size. However, in specific cases background interactions can even pose a substantial problem in a biosensor alone.

The coupling of biological molecules to a surface requires several conditions to be met. To allow for the screening of biologically relevant interactions, the biological molecules typically need to be in native (biologically active) configuration. When directly adsorbed or covalently attached, the danger of denaturation is relatively high. Therefore, they are often immobilized by long, flexible linkers or even attached to three-dimensional hydrogel matrices, which resemble physiological conditions best. The function of this matrix is manifold: first, it allows for a choice of environment conditions similar to their natural conditions, thus ensuring their biological functionality is preserved. Moreover, an appropriate matrix is capable of reducing the non-specific adsorption of undesired species to the surface and, in addition, due its three-dimensional structure offers more binding sites than a flat surface resulting in an improved signal-to-noise ratio. This combination of properties makes immobilization matrices comprising the above mentioned matrices suited for, for instance, biosensing.

Biomolecules may be linked to the matrix through electrostatic interaction, however, preferably they are linked through covalent bonds so as to increase robustness to varying experimental conditions and inadvertent loss of biological molecule due to leaching. The covalent attachment of biomolecules to such a matrix is often done by means of the amine coupling reaction. However, other coupling reactions are also used. To increase the amount of immobilized biological molecules several parameters can be optimised, such as the structure of the matrix and the procedure of immobilisation. To facilitate the immobilisation of biological molecules to the matrix it is preferred to concentrate them near the site of coupling prior or during the coupling reaction. A preferred way of concentrating biological molecules near the site of coupling is through electrostatic interaction. Biological molecules typically exhibit a net charge under certain conditions, the pH being the determining factor in this. This charge is used in some instances to concentrate biological molecules to the site of coupling. This is typically done by using a matrix containing groups bearing an opposite charge under the selected conditions. Procedures allowing for the electrostatic pre-concentration of the biological molecules in the matrix, or close to the matrix as a result of electrostatic interactions, have proven to be especially useful. In US 5,436,161, matrices in the form of hydrogels suitable to fulfil this task are described. In WO 92/06380, the conditions for coupling biological molecules to such a hydrogel are specified. A problem with the approaches listed in the cited art, is that the charges used for electrostatic pre-concentration remain present after the immobilisation step. Those charges may interfere with the subsequent analysis of biological samples, causing for instance the analyte molecules to electrostatically adsorb to the matrix instead of interacting specifically with the covalently immobilised capture probes.

The present invention provides a matrix for coupling a biological molecule thereto, said matrix comprising a polymer that is activated to contain a charged group for concentrating said biological molecule carrying an opposite charge and a reactive leaving group for covalently linking said biological molecule to said polymer, characterized in that said polymer essentially only comprises charges that are associated with said leaving group. By having the charge on the reactive leaving group, the charge is present as long as no biological molecule is coupled to the polymer at the site of the charge. The charge therefore functions to pre-concentrate the biological molecule near the reactive site. Upon covalent linking of the biological molecule, the reactive leaving group dissociates from the polymer thereby removing the charge from the polymer. Having the charge on the reactive leaving group thus serves the purpose of pre-concentration while simultaneously ensuring that no charge is left subsequent to the coupling of the biological molecule, thus avoiding undesired interference of the charge in subsequent manipulations of the matrix. Another advantage of having the charge on the reactive leaving group is that biological molecules are pre-concentrated at sites that actually comprise a reactive leaving group. In the art, where the charge is not associated with the leaving group, unequal distribution of reactive leaving groups and charges can have the result that at least some of the pre-concentrated biological molecules are not in the vicinity of a reactive leaving group. The present invention ensures pre-concentration at reactive sites thereby increasing the overall efficiency of coupling. Of course pre-concentration is particularly useful for biological molecules that are in limited quantities. Thus, preferably said biological molecule is present in concentrations between 10⁻¹¹ mol/l and 10⁻ ⁶ mol/l.

In a preferred embodiment employing sulphate or sulphonate groups on the reactive leaving group, the invention allows for an efficient coupling of biological molecules (in particular proteins) exhibiting a low pI, which is difficult with a state of the art method [O'Shannessy, Analytical Biochemistry 205, 132-136 (1992), remark on page 132] and at least costly and labour intensive with some more elaborated alternative immobilisation methods [O'Shannessy, Analytical Biochemistry 205, 132-136 (1992)]. In a preferred embodiment the present invention utilises charged groups with a negative . charge under the conditions of coupling. Having a negative charge allows the pre-concentration to be performed under conditions suitable for preconcentrating a large variety of biological molecules.

In a preferred embodiment the matrix comprises a hydrogel, said hydrogel comprising a collection of polymers according to the invention. Hydrogels have the advantage that the hydrogel is capable of reducing the non-specific adsorption of undesired species to the surface thereby improving the signal to noise ratio of assays performed with the matrix. Preferably, said matrix is three dimensional. A three-dimensional structure offers more binding sites than a flat surface resulting in an improved signal-to-noise ratio. Moreover, three-dimensional polymer matrices are more effective in the suppression of non-specific adsorption.

Preferably said hydrogel consists of a collection of polymers according to the invention. Although the invention may be favourably worked with matrices that, apart from the polymers of the invention, also comprise polymers with a charge that is not associated with a reactive leaving group, the full advantage is only obtained using matrices that consist predominantly and preferably only of polymers according to the invention.

Many different polymers are suited for the present invention. Preferably, a polymer, used to couple a reactive group to, comprises a polysaccharide, a polyamide, or a synthetic polyhydroxy compound such as poly(vinyl) alcohol. Of course, to couple the reactive leaving group the polymers first need to be chemically modified if they don't comprise suitable groups for coupling of the reactive group. Modification of polymers can be achieved in various ways. Preferably, modification of polymers is achieved using the means and methods disclosed in [P. Cuatrecasas et al., Biochemistry 11,2291-2299 (1972) or S. Löfås, B. Johnsson, J. Chem. Soc., Chem. Commun.: 1526 - 1528, 1990]. Reactive leaving groups may be coupled to modified polymers using a variety of different methods. Preferably, reactive leaving groups are coupled to a suitable polymer by means and methods described in [O'Shannessy, Analytical Biochemistry 205, 132-136 (1992) and Löfås et al., Biosensors & Bioelectronics 10, 813-822 (1995) and Lahiri et al., Analytical Chemistry 71, 777-790 (1999)]. Said reactive leaving group may be a biological molecule itself. Preferably, said leaving group is coupled to the matrix by intermediate activation through N-ethyl-N'-(3-dimethylaminopropyl)carbodiimide (EDC). In a preferred embodiment said reactive leaving group enables an amine coupling reaction for covalently linking of said biological molecule to said polymer. Amine coupling reactions are very versatile and can be used to couple a wide variety of biological molecules. In a preferred embodiment said reactive leaving group comprises a structure of formula (I), (II) or (III) 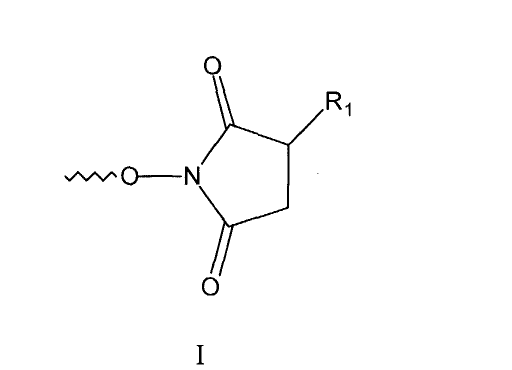 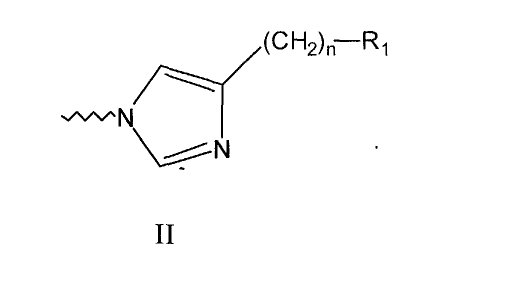 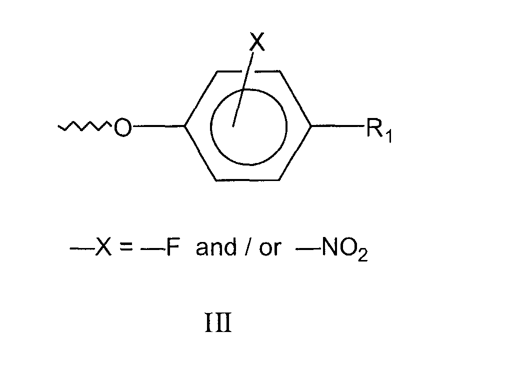 wherein R1 comprises said charge.

In principle any group comprising a negative charge under the elected reaction conditions can be used on the reactive leaving group. Pre-concentration will be achieved by the negative charge under these conditions. Preferably, said charged group comprises at least two atoms, and more preferably at least three atoms that are negatively charged under the elected reaction conditions. Preferably, said charged group is derived from a strong acid. Preferably, said leaving group comprises a sulphate, a sulphonate, a phosphate, or a phosphonate, or a combination thereof. More preferably, said leaving group comprises a sulphate or a sulphonate group since being rests of strong acids they remain deprotonated at low pH value. A sulphate or sulphonate as a charge on the reactive leaving group at least in part facilitates the selection of a suitable pH for every specific protein and every specific hydrogel. For many proteins - those not exhibiting extreme pI values - there is no need of choosing different pH conditions, even if carboxy groups are employed for the electrostatic preconcentration. However, if e. g. carboxy groups are attached to the leaving groups, it will not at all be possible to find suitable conditions for electrostatic preconcentration for some proteins. This disadvantage can be avoided by using a group comprising a rest of a strong acid, preferably a sulphate group and/or a sulphonate group. The choice of the pH conditions is dependent on the pI of the biomolecule to be immobilised and the preconcentrating group. Reactive leaving groups and conditions can be chosen such that in principle any biological molecule can be coupled to a matrix of the invention.

Biological molecules may comprise for instance a steroid, a hormone, a fatty acid, a proteinacous molecule, an oligosaccharide and other biological molecules. With the term biological molecule as used herein is meant a molecule that in principle can be made using biological techniques. It is not meant to refer to only molecules that actually are made using a biological technique. Purely synthetic molecules that can be made in a biological system are considered equivalent to a biological molecule as used herein and may also be used in the present invention instead of, or next to a biological molecule as defined herein. Non-limiting examples such equivalents that are also encompassed in the present invention are organic compounds such as steroids, peptides and nucleic acids that are synthesised chemically. Such molecules are for the present invention suitable equivalents of biological molecules as defined herein even if they do not have a counterpart in nature. A derivative of a biological molecule or equivalent thereof as defined above can also be used to work the present invention. Derivatives may include chemical modifications of biological molecules or equivalents thereof as defined above. Such chemical modifications may be introduced, for instance, to provide an (additional) group for coupling to a polymer of the invention or to shield undesired activities from the agents added. In a preferred embodiment said biological molecule or equivalent or derivative thereof comprises a proteinaceous molecule or a nucleic acid molecule, or a combination thereof. A proteinaceous molecule comprises at least two amino-acids linked to each other with a peptide bond. It preferably further comprises additional amino-acids linked with peptide bonds. A proteinaceous molecule may comprise a modification such as typically added in post-translational modification reactions of proteins. Non-limiting examples are glycosylation and/or myristilisation. One or more of the amino acids may also comprise a chemical modification. A nucleic acid may be RNA, DNA, single stranded or double stranded. It may also comprise analogues of nucleic acid such as PNA.

A surface of the invention can in principle consist of any surface as long as it does not interfere with reactions to be performed. Preferably, the surface is inert under reaction conditions chosen. In a preferred embodiment said surface comprises a metal, especially a noble metal, glass, quartz, silicon, a semiconductor, a metal oxide, a plastic or a combination thereof. In a preferred embodiment the surface is covalently linked to a hydrogel of the invention. Preferably, through covalent linkage of at least one polymer of said matrix.

In another aspect the invention provides a method for producing a matrix according to the invention, comprising providing a polymer, activating said polymer to contain a charge and a reactive leaving group, characterized in that said polymer is activated with a reactive leaving group comprising said charge. Preferably, said leaving group is a leaving group resulting from activation through N-ethyl-N'-(3-dimethylaminopropyl)carbodiimide (EDC). EDC is only used to facilitate the coupling of suitable leaving groups to the matrix, and typically forms only short lived intermediates. EDC is not a molecule suited as a leaving group itself. Activation of the polymer is preferably achieved using means and methods described in [P. Cuatrecasas et al., Biochemistry 11.2291-2299 (1972) or S. Löfås, B. Johnsson, J. Chem. Soc., Chem. Commun.: 1526-1528, 1990]. In a preferred embodiment the step comprising providing the polymer with the leaving group is performed to "near" completion such that the number of charged groups on the polymer that are not associated with a leaving group is minimized. Typically, this can be achieved by sufficiently long reaction times and sufficiently high reagent concentrations. E. g., to convert carboxymethyl groups into sulpho-NHS esters, 23 mg of EDC and 22 mg of sulpho-NHS in 300 µl of water for 20 minutes are applied.

In another aspect the invention provides a method for coupling a biological molecule to a polymer in a matrix, comprising contacting said biological molecule with a matrix according to the invention, and optionally deactivating unreacted reactive leaving groups. In a preferred embodiment said contacting of a biological molecule is performed as a separate step. When pre-concentration is desired, conditions can be chosen to ensure that said biological molecule comprises a charge opposite to the charged group on said leaving group. Suitable conditions for the coupling of various types of biological molecules are disclosed in [Löfås et al, Analytical Biochemistry 198, 268-277 (1991)]. If sulphate or sulphonate groups are present in the leaving groups, lower pH values can be chosen to couple biomolecules with low pI values, thus ensuring an effective electrostatic preconcentration for those biomolecules. Typically, unreacted leaving groups are deactivated in a deactivating step using ethanolamine. However, other deactivators such as alpha-amino-omega-hydroxy-oligo(ethyleneglycols) with a short chain length, typically between 3 and 10 repeat units, can also be used. Although it is typically preferred to-deactivate remaining reactive leaving groups, it is possible that the step comprising the coupling of biological molecule is performed such that "nearly" all reactive leaving groups have been removed by said coupling. A minor amount of reactive leaving group in the matrix after coupling of the biological molecule can be tolerated for some purposes. Preferably, however, unreacted leaving groups are deactivated. This is typically done in situations where maximal profit from the robustness and/or inertness of the matrix of the invention to the large amount of biological interactions that may occur, is desired, such as for instance in most microarray settings.

The invention further provides a matrix comprising a biological molecule obtainable by a method of the invention. Preferably, said matrix is characterised in that polymer in said matrix is essentially free of charged groups.

A matrix of the invention may be directly associated with a surface or via one or more interlayers. Suitable interlayers are known in the art. Some suitable interlayers are listed in EP 0203767.9, DE 198 17 180.3 or EP 589867. Suitable interlayers comprise the property of efficiently insulating the biosensing coating from the original surface of the substrate.

The invention further provides a surface comprising a matrix of the invention. Preferably, said surface comprises a plurality of matrices of the invention. More preferably, said plurality of matrices are arranged in a microarray. In another aspect the invention provides the use of a matrix of the invention or a surface comprising a matrix of the invention for detecting in a sample, a binding partner of a biological molecule linked to said matrix and/or surface. In yet another aspect the invention provides the use of a surface comprising a plurality of matrices according to the invention, for detecting in a sample, a binding partner of a biological molecule linked to a matrix of said plurality of matrices. Preferred is a use wherein at least one binding property of said biological molecule is known. In preferred aspect of this embodiment said biological molecule comprises an antibody or a functional part, derivative and/or analogue thereof. This embodiment is particularly useful for determining whether a test sample comprises a binding partner of said antibody. Antibody arrays are useful for a large variety of different applications some which are listed in WO 99/39210 A1. In a preferred embodiment a matrix of the invention is used for identifying said binding partner in said sample.

The interaction of a biological molecule with a test agent in a matrix of the invention can be detected by fluorescence [Daniel W. Chan, General Principle of Immunoassay, p. 1-9, in: Immunoassay - A Practical Guide, Academic Press, San Diego, California (1995)] or in various other ways, some of which are listed in [Gauglitz et al., Biosensors & Bioelectronics 10, 923-936 (1995)]. Interactions may be stacked (so-called sandwich assays), i.e. a first agent interacts with the biological molecule whereupon one or more other agents may interact with another or the same part of the biological molecule. Interaction can also comprise so-called stacked interactions wherein further biological molecules interact with biological molecules coupled previously to the matrix.

In another embodiment the invention provides a use of the invention for identifying a binding property of said biological molecule. This use has practical implications in for instance the identification of the binding potential of a biological molecule. Knowledge of the binding potential of a biological molecule is of interest for determining for instance the binding potential of an antibody or functional equivalent of derivative thereof. This has application in proteomics where antibody arrays are used to analyse at least part of the entire proteome of an organism. In such applications it is important that the full binding potential of each antibody is at least characterised.

In another aspect the invention provides the use of a matrix or a surface of the invention as a biosensor. Some of such uses are disclosed in [Gauglitz et al., Biosensors & Bioelectronics 10 ,923-936 (1995)]. Definitions of a biosensor are given e. g. in [M. P. Byfield and R. A. Abuknesha, Biosensors & Bioelectronics 9, 373-400 (1994)] or in [R. S. Sethi, Biosensors & Bioelectronics 9, 243-264 (1994)]. The invention further provides the use of a matrix or a surface of the invention in proteomics.

### Brief description of the drawings

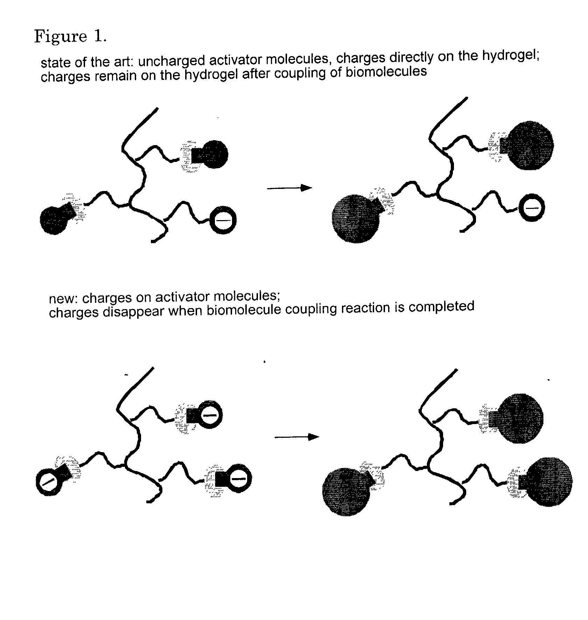

Figure 1. Schematic representation of the structure of a prior art matrix and a matrix of the invention.

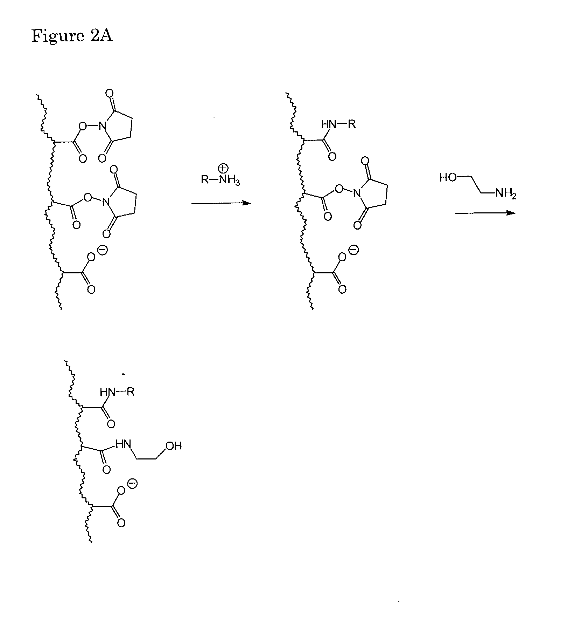

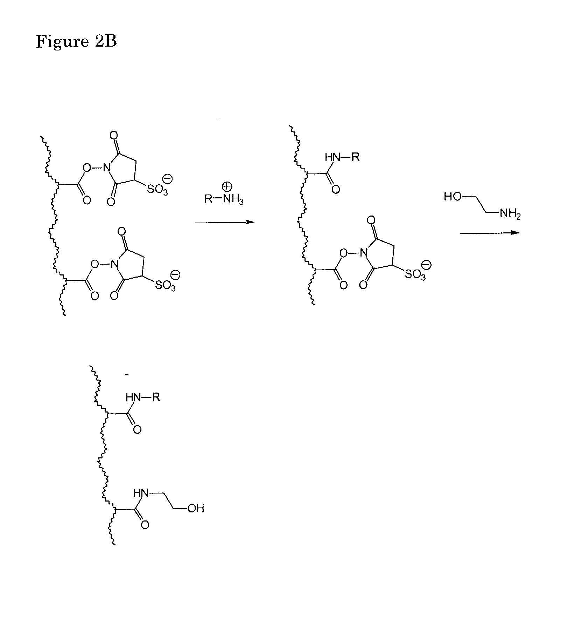

Figure 2. Schematic representation of the coupling of a biological molecule to a matrix of the art (A) and a matrix of the invention (B).

### Experimental

### Comparative study on a conventionally activated matrix coating and a matrix coating according to the invention

### 1. Preparation of two identical hydrogel coatings involving the following steps:

(1) cleaning of gold surface by immersion in 0.1M KOH/ 30wt% H₂O₂ (50:50 vol.) for 20 minutes at 60°C
(2) immersion of cleaned gold surface in a solution of thioctic acid (2 · 10⁻² mol ·1⁻¹) in water for 20 h at ambient temperature
(3) rinsing with water for 1 minute
(4) incubation with 23 mg of EDC and 13 mg of sulfo-NHS in 300 µl of water for 20 minutes
(5) rinsing with water for 1 minute
(6) incubation with 5 weight% poly(allylamine hydrochloride) in H2O for 20 minutes
(7) rinsing with water for 1 minute
(8) incubation with a mixture of 500 µl of a poly(acrylic acid) solution (2 • 10⁻² mol ·1⁻¹), 10 mg of EDC and 10 mg of sulfo-NHS
(9) rinsing with water for 1 minute
(10) incubation with 5 weight% poly(allylamine hydrochloride) in H₂O for 20 minutes
(11) rinsing with water for 1 minute
(12) incubation with mixture of a 2 wt% solution of carboxymethyldextran in H₂O with 18 mg of EDC and 18 mg of sulfo-NHS for 20 minutes
(13) rinsing with water three times for 1 minute

### Example A: Effect on the immobilisation efficiency of a medium pI protein (protein A, pI = 5.1, a protein from staphylococcus aureus that specifically binds to the F_{c} parts of immunglobuline G (IgG))

### 2. Covalent immobilisation of protein A to the multilayer assembly

### a. Conventional method

(1) incubation with a solution of 11 mg of EDC and 20 mg of NHS in 300 µl of water for 7 minutes
(2) incubation with a 100 µg/ml solution of protein A in sodium acetate buffer pH 4.7 (c= 10 mmol/l) for 20 minutes
(3) rinsing with sodium acetate buffer pH 4.7 for 30 s
(4) incubation with a solution of 0.98 g of ethanolamine in 10 ml of water pH 8.5 for 10 minutes
(5) rinsing with sodium acetate buffer pH 4.7 for 1 minute

### b. New method

(1) incubation with a solution of 11 mg of EDC and 22 mg of sulfo-NHS in 300 µl of water for 20 minutes
(2) incubation with a 100 µg/ml solution of protein A in sodium acetate buffer pH 4.7 (c= 10 mmol/l) for 20 minutes
(3) rinsing with sodium acetate buffer pH 4.7 for 30 s
(4) incubation with a solution of 0.98 g of ethanolamine in 10 ml of water pH 8.5 for 10 minutes
(5) rinsing with sodium acetate buffer pH 4.7 for 1 minute
   Result: increased efficiency of immobilisation (higher quantity of protein A bound) with new method, non-specific adsorption due to electrostatic interaction in the final assembly lowered

### Example B: Effect on the immobilisation efficiency of a low pI protein (glucose oxidase, pI = 4.15)

### 2. Covalent immobilisation of glucose oxidase to the multilayer assembly

### a. Conventional method

(1) incubation with a solution of 11 mg of EDC and 20 mg of NHS in 300 µl of water for 7 minutes
(2) incubation with a 100 µg/ml solution of glucose oxidase in sodium acetate buffer pH 3.7 (c= 10 mmol/l) for 20 minutes
(3) rinsing with sodium acetate buffer pH 3.7 for 30 s
(4) incubation with a solution of 0.98 g of ethanolamine in 10 ml of water pH 8.5 for 10 minutes
(5) rinsing with sodium acetate buffer pH 3.7 for 1 minute

### b. New method

(1) incubation with a solution of 11 mg of EDC and 22 mg of sulfo-NHS in 300 µl of water for 20 minutes
(2) incubation with a 100 µg/ml solution of glucose oxidase in sodium acetate buffer pH 3.7 (c= 10 mmol/l) for 20 minutes
(3) rinsing with sodium acetate buffer pH 3.7 for 30 s
(4) incubation with a solution of 0.98 g of ethanolamine in 10 ml of water pH 8.5 for 10 minutes
(5) rinsing with saline sodium acetate buffer pH 3.7 for 1 minute
   Result: Strongly increased efficiency of immobilisation (higher quantity of glucose oxidase bound) with new method, non-specific adsorption due to electrostatic interaction in the final assembly lowered

## Claims

1. A matrix for coupling a biological molecule thereto, said matrix comprising a polymer that is activated to contain a charged group for concentrating said biological molecule carrying an opposite charge and a reactive leaving group for covalently linking said biological molecule to said polymer, **characterised in that** said polymer essentially only comprises charges that are associated with said leaving group.

2. A matrix according to claim 1, wherein said charged group comprises a negative charge.

3. A matrix comprising a hydrogel, said hydrogel comprising a collection of polymers according to claim 1 or claim 2.

4. A matrix according to claim 3, wherein said hydrogel consists of a collection of polymers according to claim 1 or claim 2.

5. A matrix according to any one of claims 1-4, wherein essentially each polymer of said collection is associated with a surface.

6. A matrix according to any one of claims 1-5, wherein said polymer comprises a polysaccharide, a polyamide, or a synthetic polyhydroxy compound such as poly(vinyl) alcohol.

7. A matrix according to any one of claims 1-6, wherein said reactive leaving group enables an amine coupling reaction for covalent linkage of said biological molecule to said polymer.

8. A matrix according to any one of claims 1-7, wherein said reactive leaving group comprises a structure of formula (I), (II) or (III) 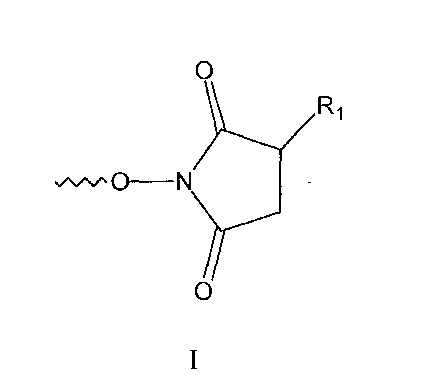 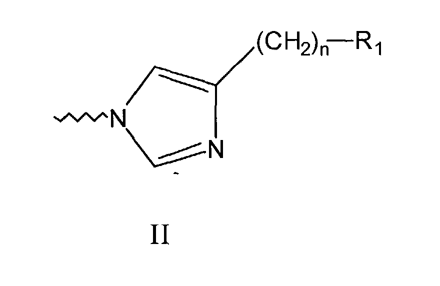 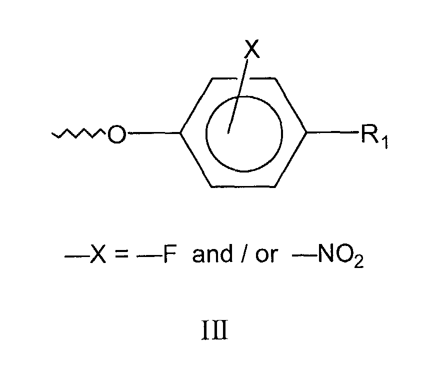 wherein R₁ comprises said charge.

9. A matrix according to claim 7 or claim 8, wherein said charged group comprises a sulphate, a sulphonate, a phosphate, or a phosphonate, or a combination thereof.

10. A matrix according to any one of claims 1-9, wherein said biological molecule comprises a proteinaceous molecule or a nucleic acid molecule, or a combination thereof.

11. A matrix according to any one of claims 5-10, wherein said matrix is three dimensional.

12. A method for producing a matrix according to any one of claims 1-11, comprising providing a polymer, activating said polymer to contain a charge and a reactive leaving group, **characterized in that** said polymer is activated with a reactive leaving group comprising said charge.

13. A method for coupling a biological molecule to a polymer in a matrix, comprising contacting said biological molecule with a matrix according to any one of claims 1-11, and optionally deactivating unreacted reactive leaving groups.

14. A method according to claim 13, wherein said coupling is performed under conditions allowing pre-concentration of said biological molecule in said matrix.

15. A matrix comprising a biological molecule obtainable by a method according to claim 13 or claim 14.

16. A matrix according to claim 15, **characterized in that** polymer in said matrix is essentially free of charged groups.

17. Use of a matrix according to claim 15 or claim 16, and/or a surface comprising said matrix, for detecting in a sample, a binding partner of a biological molecule linked to said matrix and/or surface.

18. A surface comprising a matrix according to any one of claims 1-11, 15 and 16.

19. A surface according to claim 18 comprising a plurality of matrices according to any one of claims 1-11, 15 and 16.

20. A surface according to claim 19, wherein said plurality of matrices are arranged in a microarray format.

21. Use of a surface according to claim 19 or claim 20 comprising a-plurality of matrices according to claim 15 or claim 16 for detecting a binding partner in a sample, where this binding partner is a binding partner of a biological molecule linked to a matrix within said plurality of matrices.

22. Use according to claim 21 wherein at least one binding property of said biological molecule is known.

23. Use according to any one of claims 17, 21 or 22, for identifying said binding partner in said sample.

24. Use according to any one of claims 17, 21 or 22, for identifying a binding property of said biological molecule.

25. Use of a matrix according to claim 15 or claim 16, and/or a surface according to any one of claims 18-20, as a biosensor.

26. Use of a matrix according to claim 15 or claim 16, and/or a surface according to any one of claims 18-20, in proteomics.
